# EUROPEAN PATENT SPECIFICATION

(11) **EP 1 638 496 B1**
(45) Date of publication and mention of the grant of the patent: **08.05.2013**
(21) Application number: 04756496.8
(22) Date of filing: 30.06.2004
(51) Int. Cl.: A61F 13/15, D01D 5/42, D04H 13/02

(54) **ARTICLES CONTAINING NANOFIBERS PRODUCED FROM A LOW ENERGY PROCESS**
ARTIKEL MIT NANOFASERN, DIE IN EINEM VERFAHREN MIT NIEDRIGER ENERGIE HERGESTELLT WURDEN
ARTICLES CONTENANT DES NANOFIBRES PRODUITES A PARTIR D'UN TRAITEMENT A FAIBLE ENERGIE

(30) Priority: 30.06.2003 US 483729 P
(43) Date of publication of application: 29.03.2006
(73) Proprietor: THE PROCTER & GAMBLE COMPANY, Cincinnati, OH 45202 (US)
(72) Inventor: ISELE, Olaf, Erik, Alexander, West Chester, OH 45069 (US); CHHABRA, Rajeev, Mason, OH 45040 (US)
(74) Representative: Borbach, Markus
(86) International application number: PCT/US2004/021129
(87) International publication number: WO 2005/004768

(56) References cited:
- WO-A-97/05306
- US-A- 3 806 289
- US-A- 4 100 324
- US-A- 4 587 154
- US-A- 4 818 464
- RENEKER D H: "NANOFIBERS FOR ENGINEERD TEXTILES" UMIST CONFERENCE. TEXTILES ENGINEERED FOR PERFORMANCE, XX, XX, 20 April 1998 (1998-04-20), page COMPLETE, XP002919296

## Description

### FIELD OF THE INVENTION

The present invention relates to hygiene articles made from nanofibers and method of producing the nanofibers from a low energy process.

### BACKGROUND OF THE INVENTION

The need for articles produced from nonwoven containing nanofibers has continued to increase. The diameters of nanofibers are generally understood to be less than about 1000 nanometer or one micron. The nanofibers webs are desired due to their high surface area, low pore size, and other characteristics. The nanofibers, also commonly called microfibers or very fine fibers, can be produced by a variety of methods and from a variety of materials. Although several methods have been used, there are drawbacks to each of the methods and producing cost effective nanofibers has been difficult. Therefore, hygiene articles and other disposable consumer products containing nanofibers have not been marketed.

Methods of producing nanofibers include a class of methods described by melt fibrillation. Melt fibrillation is a general class of making fibers defined in that one or more polymers are molten and extruded into many possible configurations, such as co-extrusion, homogeneous or bicomponent films or filaments, and then fibrillated or fiberized into fibers. Nonlimiting examples of melt fibrillation methods include melt blowing, melt film fibrillation, and melt fiber bursting. Methods of producing nanofibers, not from melts, are film fibrillation, electro-spinning, and solution spinning. Other methods of producing nanofibers include spinning a larger diameter bicomponent fiber in an islands-in-the-sea, segmented pie, or other configuration where the fiber is further processed after the fiber has solidified so that nanofibers result.

Melt blowing is a commonly used method of producing fibers. Typical fiber diameters range from 2 to 8 micron. Melt blowing can be used to make fibers with smaller diameters but with considerable changes needed to the process. Commonly, redesigned nozzles and dies are needed. Examples of these include U.S. Patent Nos. 5,679,379 and 6,114,017 by Fabbricante et al. and U.S. Patent Nos. 5,260,003 and 5,114,631 by Nyssen et al. These methods utilize relatively high pressures, temperatures, and velocities to achieve the small fiber diameter.

Melt film fibrillation is another method to produce fibers. A melt film tube is produced from the melt and then a fluid is used to form nanofibers from the film tube. Two examples of this method include Torobin's U.S. Patent Nos. 6,315,806; 5,183,670; and 4,536,361; and Reneker's U.S. Patent Nos. 6,382,526 and 6,520,425, assigned to the University of Akron. Although these methods are similar by first forming a melt film tube before the nanofibers result, the processes use different temperatures, flow rates, pressures, and equipment.

Film fibrillation is another method of producing nanofibers although not designed for the production of polymeric nanofibers to be used in nonwoven webs. U.S. Patent No. 6,110,588 by Perez et al., assigned to 3M, describes of method of imparting fluid energy to a surface of a highly oriented, highly crystalline, melt-processed polymer film to form nanofibers. The films and fibers are useful for high strength applications such as reinforcement fibers for polymers or cast building materials such as concrete.

Electrospinning is a commonly used method of producing nanofibers. In this method, a polymer is dissolved in a solvent and placed in a chamber sealed at one end with a small opening in a necked down portion at the other end. A high voltage potential is then applied between the polymer solution and a collector near the open end of the chamber. The production rates of this process are very slow and fibers are typically produced in small quantities. Another spinning technique for producing nanofibers is solution or flash spinning which utilizes a solvent.

Two-step methods of producing nanofibers are also known. A two-step method is defined as a method of forming fibers in which a second step occurs after the average temperature across the fiber is at a temperature significantly below the melting point temperature of the polymer contained in the fiber. Typically, the fibers will be solidified or mostly solidified. The first step is to spin a larger diameter multicomponent fiber in an islands-in-the-sea, segmented pie, or other configuration. The larger diameter multicomponent fiber is then split or the sea is dissolved so that nanofibers result in the second step. For example, U.S. Patent Nos. 5,290,626 by Nishio et al., assigned to Chisso, and 5,935,883, by Pike et al., assigned to Kimberly-Clark, describe the islands-in-the-sea and segmented pie methods respectively. These processes involve two sequential steps, making the fibers and dividing the fibers.

To produce disposable hygiene articles containing nanofibers that are commercially advantageous, the cost of the nanofibers must be controlled. Equipment cost, process costs, any additional process aids, and polymer costs are all areas where costs can be controlled. Therefore, it is an object of the invention to produce nanofibers which are low in cost.

One method of controlling costs is to have a low energy process.

### SUMMARY OF THE INVENTION

The present invention is directed to hygiene articles comprising nanofibers. The nanofibers are made from an energy efficient melt fibrillation process. The melt fibrillation process will have a polymer throughput of more than about 10 grams per minute per orifice. Preferably, the process will have a fiberizing fluid temperature of less than about 50°C above the polymer melting point and a fiberizing fluid velocity of less than about 100 meters per second. The nanofibers, having a diameter of less than 1 micron, must comprise a significant number of the fibers in one layer of the web contained by the hygiene article. Preferably, the nanofibers are produced in a melt film fibrillation process. The hygiene articles include diapers, training pants, adult incontinence pads, catamenial products such as feminine care pads and pantiliners, tampons, personal cleansing articles, personal care articles, and personal care wipes including baby wipes, facial wipes, and feminine wipes.

### DETAILED DESCRIPTION OF THE INVENTION

The present invention relates to hygiene articles made from nanofibers. The nanofibers are produced from one or more thermoplastic polymers. Nonlimiting examples of thermoplastic polymers suitable for the present invention include polyolefins, polyesters, polyamides, polystyrenes, polyurethanes, biodegradable polymers including thermoplastic starch, PHA, PLA, starch compositions, and combinations thereof. The homopolymer, copolymers, and blends thereof are included within this description. The most preferred polymers are polyolefins such as polypropylene, polyethylene, nylons, and polyethylene terephthalate.

Suitable thermoplastic polymers include any polymer suitable for melt spinning. The rheological properties of the polymer must be such that the polymer can be melt extruded and is able to form a film. The melting temperature of the polymer is generally from about 25°C to 400°C. The polymers of the present invention as they are present in the die may have a melt flow rate of less than about 400 decigrams per minute. The melt flow rate is measured using ASTM method D-1238. Preferably, the melt flow rate may be less than about 300 decigrams per minute, more preferably less than about 200 decigrams per minute, and most preferably less than about 100 decigrams per minute. A most preferred range for melt flow rates is from about 1 decigram per minute to about 100 decigrams per minute. Generally, the lower the melt flow rate the more preferred. Therefore, polymers with melt flow rates less than about 50 decigrams per minute and 40 decigrams per minute may be utilized.

The fibers may be single or multicomponent fibers such as bicomponent fibers. The fibers may have a sheath-core or side-by-side or other suitable geometric configuration. After the fibers are made, the fibers may be treated or coated before formed into a web. Additionally, after a web is made, the web may be treated. Optionally, additives may be compounded into the polymer resin and these additives may move out to the surface of the fiber after the fibers are formed. The additives that migrate to the surface may need to be cured utilizing external energy, such as heat, or additives on surface may need to be chemically reacted with another component or curing may need to be catalyzed in the presence of another component, such that additional components may be added to the process while the fibers are being made or after die fibers are made using the resin with additives. Suitable treatments include hydrophilic or hydrophobic treatments. An example of hydrophobic treatment is poly-di-methyl-siloxanes. The specific treatment depends on the use of the web, type of polymer, and other factors.

Optionally, the polymer may contain additional materials to provide other properties for the fiber. These may modify the physical properties of the resulting fiber such as elasticity, strength, thermal or chemical stability, appearance, absorbency, odor absorbency, surface properties, and printability, among others. A suitable hydrophilic melt additive may be added. Optional materials may be present up to 50% of the total polymer composition.

The method of making the nanofibers of the present invention is any one-step melt fibrillation process that has a polymer throughput of more than about 10 grams per minute per orifice. Melt fibrillation processes are defined as processes utilizing a single phase polymer melt wherein fibers are formed. Single phases can include a dispersion but do not include solvent based melts such as those used in solution or electrospinning. Typical single step melt fibrillation processes include melt blowing, melt film fibrillation, spun bonding, melt spinning in a typical spin/draw process, and combination thereof. Single step processes do not include two-step processes where the fiber is first made and then split (e.g. segmented pie or islands-in-the-sea) or processed further after the fibers have solidified.

The method of making the nanofibers of the present invention is a melt fibrillation process and more preferably a melt film fibrillation process. Generally, a melt film fibrillation process involves providing a polymeric melt, utilizing a central fluid stream to form an elongated hollow polymeric film tube, and then using a fluid to form multiple nanofibers from the hollow tube. Suitable methods are detailed, for example, in U.S. Patent No. 4,536,361 to Torobin and U.S. Patent Nos. 6,382,526 and 5,520,425 to Reneker. The melt film fibrillation methods can utilize various different processing conditions. Reneker's method more specifically includes the steps of feeding the polymer into an annular column and forming a film or tube at the exit of the annular column where a gas jet space is formed. A gas column then provides pressures on the inner circumference of the polymer tube. When the polymer tube exits the gas jet space, it is blown apart into many small fibers, including nanofibers, due to the expanding central gas.

An example of a melt film fibrillation method more specifically includes the steps of melting the polymer to form a polymeric melt. The polymeric melt will contain the polymer composition and any other ingredients. The polymeric melt is extruded through an orifice which in turn contains a central fluid stream such that the polymer extrudes as an elongated hollow tube. The orifice may be part of a nozzle. It is obvious to those skilled in the art that the overall design of the nozzle may have to be optimized for process stability. Furthermore, the central fluid stream may be concentric or eccentric. A fiberizing fluid, such as a central fluid stream, is blown to form an elongated hollow tube. The fiberizing fluid will then provide pressure on the inner surface of the elongated hollow tube. Thinned wall or weakened portions may form in the hollow tube to more easily and controllably enable the formation of fibers, including nanofibers. The weakened portions may result from notches or projections located on the outer surface of the central fluid jet tube or on the inner surface of the polymer extrusion orifice. The elongated hollow polymeric film tube is then subjected to a fluid to form the nanofibers. This fluid can be the central fluid stream or an entraining fluid or any fluid stream to induce a pulsating or fluctuating pressure field and forms a multiplicity of fibers, including nanofibers. If advantageous, a nozzle providing cooling or heating fluid to the formed nanofibers may be used.

The polymer is typically heated until it forms a liquid and flows easily. The melted polymer may be at a temperature of from about 0°C to about 400°C, preferably from about 10°C to about 300°C, and more preferably from about 20°C to about 220°C. The temperature of the polymer depends on the melting point of the polymer or polymer composition. The temperature of the polymer is less than about 50°C above its melting point, preferably less than 25°C above its melting point, more preferably less than 15°C above its melting point, and just at or above its melting point or melting range. The melting point or range is measured using ISO 3146 method. The melted polymer will typically have a viscosity of from about 1 Pa-s to about 1000 Pa-s, typically from about 2 to about 200 Pa-s and more commonly from about 4 to about 100 Pa-s. These viscosities are given over a shear rate ranging from about 100 to about 100,000 per second. The melted polymer is at a pressure of about atmospheric pressure or slightly elevated.

The elongated hollow polymer tube can be circular, elliptical, irregular, or any other shape which has a hollow region. In some cases, the elongated hollow polymer tube may collapse immediately after forming. In the case of the collapsed tube, it may be preferred to have thinned walls or weakened portions in the tube to aid in the fibrillation. Non-limiting examples of the fiberizing fluid are gases such as nitrogen or more preferably air. The fiberizing fluid is typically at a temperature close to the temperature of the melted polymer. The fiberizing fluid temperature may be a higher temperature than the melted polymer to help in the flow of the polymer and the formation of the hollow tube. Alternatively, the fiberizing fluid temperature can be below the melted polymer temperature to assist in the formation and solidification of the nanofibers. Preferably the fiberizing fluid temperature is less than the polymer melting point, more preferably more than 50°C below the polymer melting point, more preferably more than 100°C below the polymer melting point, or just at ambient temperature. The pressure of the fiberizing fluid is sufficient to fibrillate the nanofibers and can be slightly above the pressure of the melted polymer as it is extruded out of the orifice.

The fiberizing fluid may have a velocity of less than about 500 meter per second. Preferably, the fiberizing fluid velocity will be less than about 100 meter per second, more preferably less than about 60 meter per second, and most preferably from about 10 to about 50 meters per second. The fiberizing fluid may pulsate or may be a steady flow. Although it is critical that this fiberizing fluid is present to aid in the formation of the elongated hollow polymeric film tube, the amount of fluid in this stream may be very low.

The polymer throughput will primarily depend upon the specific polymer used, the nozzle design, and the temperature and pressure of the polymer. The polymer throughput will be more than about 10 grams per minute per orifice. Preferably, the polymer throughput will be more than about 15 grams per minute per orifice and more preferably greater than about 25 grams per minute per orifice. Most preferably, the polymer throughput is even higher and greater than about 40 grams per minute per orifice. There will likely be several orifices operating at one time which increases the total production throughput. The throughput, along with pressure, temperature, and velocity, are measured at the die orifice exit.

The fibrillation and solidification of the fibers may occur before the fibers and fluid exit the orifice. Once the elongated hollow tube exits the orifice, the nanofibers are formed. Commonly, the formation of nanofibers occurs immediately upon exiting the orifice. One or more fluid streams are used to form the multiplicity of nanofibers. The fluid stream can be the central fluid stream, an entraining fluid, or any other fluid stream. An entraining fluid can be used to induce a pulsating or fluctuating pressure field to help in forming a multiplicity of nanofibers. The entraining fluid may be provided by a transverse jet which is located to direct the flow of entraining fluid over and around the hollow elongated tube and nanofiber forming region. The entraining fluid can have a low velocity or a high velocity, such as near sonic or super sonic speeds. An entraining fluid with a low velocity will typically have a velocity of from about 1 to about 100 meter per second and preferably from about 3 to about 50 meter per second. The temperature of the entraining fluid can be the same as the above fiberizing fluid, ambient temperature, or a higher temperature, or a temperature below ambient.

An additional fluid stream, a quench or heating fluid, can also be used. This additional fluid stream is located to direct fluid into the nanofibers to cool or heat the fibers. If the additional fluid is used as a quenching fluid, it is at a temperature of from about -50°C to about 100°C and preferably from about 10°C to 40°C. If the additional fluid is used as a heating fluid, it is at a temperature of from about 40°C to 400°C and typically from about 100°C to about 250°C. Any fluid stream may contribute to the fiberization of the polymer melt and can thus generally be called fiberizing fluids.

Any of the fluid streams, including the central fluid stream, an entraining fluid, or additional fluid stream, may contain a treatment, additive, coating, or other substance or particulate for changing the surface, chemical physical, or mechanical properties of the fibers produced.

The nanofibers are laid down on a collector to form a web. The collector is typically a conveyor belt or a drum. The collector is preferably porous and vacuum may be applied to provide suction to aid fiber lay down on the collector. The distance from the orifice to the collector distance, commonly called die-to-collector distance (DCD), can be optimized for desired web properties. To reduce the amount of fiber bundling or roping, the DCD should be relatively low. This lower distance does not enable the fibers to have time to entangle, wrap around one another, or bundle. It may be desired to utilize more than one DCD used in a web, to change the DCD during production, or to have different beams with different DCDs. It may be desirable to form a web with different uniformities by changing the DCD.

After the elongated hollow film tube is formed, the tube or the nanofibers may alternatively be subject to an additional process that further promotes the formation of nanofibers. The further processing would occur immediately after formation of the elongated hollow polymeric film tube and before the nanofibers have solidified to still be considered a single step process. The additional processing can utilize one or more Laval nozzles to speed up the gas velocities to sonic and/or supersonic range. When polymer melt is exposed to such high gas velocities, it bursts into multiplicity of fine fibers. Examples of a Laval nozzle are described in Nyssen et al., US Patent No. 5,075,161, in which a method of bursting polyphenylene sulfide melt into fine filaments is disclosed. The Laval nozzle may be positioned just after the spinning nozzle when the elongated hollow polymeric film tube is produced. Alternatively, Laval nozzle could be positioned just after the nanofibers have formed to further reduce the fiber size. Polymer fibers can be produced by subjecting the polymer melt streams to drawing out and cooling to below the melt temperature by extruding them into a gaseous medium which flows essentially parallel to the polymer melt streams and attains sonic or supersonic speed. This simultaneous deformation and cooling gives rise to amorphous fine or extremely fine fibers of finite length. High speed fiber bursting minimizes the surface oxidation of the fibers. The spinning speed, melt temperature, and the position of the Laval nozzle are appropriately set to achieve only partial thermal oxidation of fine filaments at their surface.

Various processes and combination of processes can be used to make the webs of the present invention. Melt fiber bursting, as disclosed in WO 04/020722 by Sodemann et al., can be combined with melt film fibrillation of the present invention on two separate beams on a single line. Various aspects of melt fiber bursting can be incorporated into melt film fibrillation, such as producing fibers of different strengths and diameters to provide a desired combination of properties. Alternatively, aspects of melt film fibrillation can be included in other melt fibrillation processes to increase the throughput rate by utilizing a hollow elongate tube to form fibers. For example, the melt film fibrillation process of the present invention could be modified to include a Laval nozzle to aid in drawing down the fibers. Drawing down can aid in further attenuation and increase the strength of the fibers. This may be particularly preferred for high Tg polymers such as polyesters in which stress-induced crystallization happens at speeds in excess of 4000 m/min.

The nanofibers of the present invention are used to make nonwoven webs. The web is defined as the total nonwoven composite. A web may have one or several layers. A layer is the web or part of a web that is produced in a separate fiber lay down or forming step. The webs of the present invention will comprise one or more layers having a significant number of nanofibers having diameters of less than one micron. The significant number of fibers is at least about 35%, at least about 50%, or more than 75% of the total number of fibers in the layer. The web could have 100% of the fibers having a diameter of less than about one micron. The fiber diameters of the web are measured using a scanning electron microscope at a magnification of greater than about 500 times and up to about 10,000 times as needed for visual analysis. To determine if a significant numbed of fibers have diameters less than one micron, at least about 100 fibers and preferably more fibers must be measured. The measurements must occur at various regions throughout the layer. Sufficient sampling that is statistically significant must occur.

The fiber diameter of the remaining larger fibers, up to 75%, may have fiber diameters in any range. Typically, the larger fiber diameters will be just above one micron to about 10 microns.

Preferably, a significant number of fibers in a layer will have a fiber diameter of less than about 900 nanometers and more preferably from about 100 nanometers to about 900 nanometers. The fibers may have a diameter of less than 700 nanometers and from about 300 to about 900 nanometers. The preferred diameters depend upon the desired use of the web. For process and product benefits, it may be desirable in some applications to have a significant number of fibers having a diameter of less than about one micron and a significant number of fibers having a diameter of greater than about one micron. The larger fibers may trap and immobilize the nanofibers. This may help to reduce the amount of clumping or roping of the nanofibers and prevents the nanofibers from being carried off by stray air currents.

The layers of nanofibers in a web of the present invention may contain more than one polymer. Different polymers or polymer blends may be used for different orifices to produce layers in a web having different fiber diameters and different polymer compositions.

It may be desirable to produce a single layer nonwoven with varying fiber diameters. Alternatively, it can be desired to produce a nonwoven web with multiple layers with each layer having different fiber diameters. The melt film fibrillation process can be modified to produce both small and large diameter fibers to make various webs. The smaller fiber diameters are referred to as having a significant number of fibers having a diameter of less than one micron. The larger diameter fibers include fibers from the melt blowing range (typically 3 to 5 microns) to the spunbond (typically around 15 microns) or any range of fiber diameters above 1 micron. For example, one layer can be produced with an average fiber diameter of less than one micron and another layer with an average fiber diameter of around 5 microns. This type of structure could be used where traditionally SMS webs are used. Another example is to produce a nanofiber web with multiple layers, with each layer having a distinct average fiber diameter such as one layer having an average fiber diameter of 0.4 micron and a second layer having an average fiber diameter of 0.8 micron. The webs with various fiber diameters can be produced on the same line with the same equipment. This is an inexpensive way as the same equipment and components can be used. The operating costs and equipment costs are both controlled. Also, if desired, the same polymer can be used to produce different fiber diameters.

It may be desired to form a web of several layers. The nanofiber layer may be combined with one, two or more layers. A spunbond-nanofiber-spunbond web is one example. Basis weights for the total composite webs range from about 5 gsm to about 100, preferably from about 10 to about 100 gsm, and more preferably from about 10 gsm to about 50 gsm. For use as a barrier layers, the total composite web basis weight may be from about 10 gsm to about 30 gsm. The basis weight of only the nanofiber layer is from about 0.5 gsm to about 30 gsm and preferably from about 1 gsm to about 15 gsm.

After the nonwoven is made, it may be subject to post processing. Nonlimiting examples of post processing include solid state formation, consolidation, lamination, surface coating, corona and plasma treatment, dyeing, and printing. Nonlimiting examples of solid state formation include processes using intermeshing rolls, such as in U.S. Pat. No. 5,518,801 and referred to in subsequent patent literature as "SELF" webs, which stands for "Structural Elastic-like Film", texturing, stretching, aperturing, laminating, local straining, micro creping, hydroforming, and vacuum forming. Nonlimiting examples of consolidation include thermal bonding, through air bonding, adhesive bonding, and hydroentangling.

The hygiene articles of the present invention will contain the above described nonwoven webs The web may comprise the entire hygiene articles, such as a wipe, or the web may comprise one component of the hygiene article, such as a diaper. The hygiene articles include diapers, training pants, adult incontinence pads, catamenials products such as feminine care pads and pantiliners, tampons, personal cleansing articles, personal care articles, and hygiene wipes including baby wipes, facial wipes, body wipes, and feminine wipes. Personal care articles include articles such as wound dressings, active delivery wraps or patches, and other substrates that are applied to the body, particularly the skin.

In a diaper, the web may be used as a barrier layer or an outercover. The webs may also be used as a high barrier cuff with a high hydrostatic head to enable low leakage incident rates of thin, narrow crotch diapers desired for comfort and fit. The webs may be used in wipes to enable improved lotion handling and reduced gradient of liquids. The webs may also provide controlled delivery of a substance. The delivered substance can be of liquids, lotions, actives, or other materials. Due to the high surface area of the nanofibers, the webs may be used as absorbent materials for wipes or cores of feminine care product pads, diapers, training pants, or adult incontinence. The webs may provide enhanced distribution of fluids and/or retention. Additionally, the webs for absorbent uses may be made with added particulates or absorbent or natural fibers for increased absorbance or certain layers of the webs may have different properties. The nanofiber webs may also be used in articles wherein opaqueness is desired. Added opaqueness may result due to the small fiber diameter and uniformity; or pigments may be added to the polymer melt or webs. Commonly, the nanofiber layer is combined in a web with a spunbond layer. There may be one spunbond layer or a spunbond layer on each side of the nanofiber web.

In a diaper or other disposable absorbent product, the nonwoven web containing nanofibers may be utilized as a barrier layer. The barrier layer may be disposed between an absorbent core and an outer layer of the disposable absorbent product. The absorbent core is the component of the article that is primarily responsible for fluid handling properties such as acquiring, transporting, distributing, and storing body fluids. The absorbent core is typically located between a liquid pervious body-side inner layer and a vapor permeable, liquid impermeable outer cover. The outer layer, also known as the back sheet or outer covering, is located on the outside of the disposable product. In the case of a diaper, the outer layer contact the user's garment or clothing. The barrier layer may alternatively or also be disposed between the absorbent core and an inner layer. The inner layer, also known as a top sheet, is located on the side closest to the user's skin. The inner layer may contact the user's skin or may contact a separate top sheet with contacts the user's skin. The barrier layer may be absorbent. The nonwoven web may comprise the layer around the absorbent core and help to distribute or handle fluids. The nonwoven web may be a fluid distribution layer, which may be located adjacent to the core. The barrier layer most preferably has a balance between convective air flow and absorptive barrier property. The convective air flow property is effective to reduce the relative humidity within the space between the absorbent article and the wearer's skin. The combination of liquid absorption and liquid barrier property provides protection against the wet through problem and is especially beneficial when the absorbent article is under impact and/or sustained pressure. Further description and benefits of the barrier layers may be found in WO 01/97731.

The webs may be used to make hygiene wipes that are dry or pre-moistened. The nanofiber may be used in wipes to enable improved lotion handling and reduced gradient of liquids. The nanofiber layer may provide a partial barrier and may be partially or completely liquid impervious. The webs may also provide controlled delivery of a substance or active such as a drug. The delivered substance can be liquids, lotions, enzymes, catalysts, actives, or other materials such as emollients, surfactants, wetting agents, polishes, oils, organic and inorganic solvents, pastes, gels, pigments, or dyes. The webs may provide enhanced distribution of fluids and/or retention. Additionally, the webs for absorbent uses may be made with added particulates or absorbent or natural fibers for increased absorbance or certain layers of the webs may have different properties. The nanofibers may be produced to be low density or porous nanofibers. The nanofibers may be produced with an inflatent or blowing agent.

Due to the high surface area of the nanofibers, the webs may be used as absorbent materials for wipes or cores of feminine care product pads, diapers, training pants, or adult incontinence. The high surface area also enhances cleaning and may be used in hygiene cleaning wipes. The webs may provide enhanced distribution of fluids and/or retention. Additionally, the webs for absorbent uses may be made with added particulates or absorbent or natural fibers for increased absorbance or certain layers of the webs may have different properties.

The nanofiber webs may also be used in articles wherein opaqueness is desired. Added opaqueness may result due to the small fiber diameter and uniformity; or pigments may be added to the polymer melt or webs. The webs may also be low linting resulting from longer length fibers or entangling of fibers in the web. The tensile strength of the nanofiber webs of the present invention can be greater than the tensile strength of webs with similar fiber diameters and similar basis weights made from other processes. The nanofiber webs of the present invention will be soft and may be softer than other webs with the same performance.

Other products that will benefit from a nanofiber web include a personal filter or mask such as a surgical mask. Other medical uses of webs containing nanofiber layers include surgical gowns, wound dressings, and medical barriers.

The fiber diameter can be measured using a Scanning Electronic Microscope (SEM) and image analysis software. A magnification of 500 to 10,000 is chosen such that the fibers are suitably enlarged for measurements. Image analysis software for automatic sampling of fiber diameter in the SEM picture is possible, but also a more manual procedure can be used. In general, the edge of a randomly selected fiber is sought and then measured across the width (perpendicular to fiber direction at that spot) to the opposite edge of the fiber. A scaled and calibrated image analysis tool provides the scaling to get the actual reading in mm or micrometers (µm). Several fibers are randomly selected across the sample of web in the SEM. Typically, several samples from a web are cut and tested in this manner and at least about 100 measurements are made and all data are recorded for statistic analysis. If the result is to be recorded in denier, then the following calculation needs to be made. Diameter in denier = Cross-sectional area * density * 9000m * 1000 g/kg. The cross-sectional area is π*diameter²/4. The density for PP, e.g., can be taken as 910 kg/m³. To obtain decitex (dtex), the 9000m is replaced by 10,000m.

Basis weight can be measured consistent with compendial methods ASTM D 756, ISO 536 and EDANA ERT-40.3-90. Basis weight is defined as mass per unit area, with grams per square meter (gsm) as the preferred unit. Required instruments are a scissors or a die-cutter for sample cutting and an accurate weighing device, such as a scale. A sample is cut to a total area of 100cm² per layer with an accuracy and precision of ± 0.5%. A scale or balance is needed with 0.001 g sensitivity, readable, calibrated and accurate to within 0.25% of the applied load. The samples are conditioned at 23° Celsius (± 2°C) and at a relative humidity of about 50% for 2 hours to reach equilibrium. Weigh the cut sample with 10 plies from the sample area for a total of 1000cm² =0.1 m² on an analytical balance to the nearest 0.001g and record the weight. (For samples thicker than 1mm, weighing only 1 ply is preferred but should be noted.) Calculate the basis weight by dividing the weight by the sample area (all layers tested) to give the basis weight in gsm. All data are recorded for statistic analysis.

Web uniformity can be measured through several methods. Examples of uniformity metrics include low coefficient of variation of pore diameter, basis weight, air permeability, and/or opacity. Uniformity also requires lack of fiber bundles or roping, or visible holes, or other such defects. Uniformity can be controlled by process modification such as reducing the nozzle to collector distance. The reduction in this distance reduces the fiber bundling or roping and can provide more uniform webs.

Pore diameter can be determined by methods known to those skilled in the art. The mean pore diameter is less than about 15 microns, more preferably less than about 10 microns, and most preferably less than about 5 microns. The coefficient of variation for a uniform web is less than 20%, preferably less than about 15%, and more preferably about 10% or less. The lack of roping can be measured by counting the number of ropes or bundles of fibers in a measured area of the web. The lack of holes is also measured the number of holes having a diameter above a certain threshold in a measured area of the web. The holes may be counted if they are visible to the naked eye or are more than 100 microns in diameter.

## Claims

1. A hygiene article comprising a nonwoven web comprising a layer having at least 35% nanofibers with a diameter less than one micron,
wherein said nanofibers are produced from one or more thermoplastic polymers and are made from a single step melt fibrillation process having a polymer throughput of greater than 10 grams per minute per orifice; and,
wherein the melt fibrillation process utilizes a fiberizing fluid supplied at a temperature close to, at or below the temperature of the molten polymer(s).

2. The hygiene article according to claim 1, wherein the polymer has a melt flow rate of less than 400 decigrams per minute.

3. The hygiene article according to claim 2, wherein the polymer has a melt flow rate of less than 100 decigrams per minute.

4. The hygiene article according to any one of claims 1 to 3, wherein the melt fibrillation process utilizes a fiberizing fluid supplied at a temperature less than the polymer melting point, preferably more than 50°C below the polymer melting point.

5. The hygiene article according to any one of claims 1 to 4, wherein the melt fibrillation process utilizes a fiberizing fluid supplied at ambient temperature.

6. The hygiene article according to any one of claims 1 to 5, wherein the melt fibrillation process utilizes a fiberizing fluid with a velocity of less than 100 meters per second.

7. The hygiene article according to any one of claims 1 to 6, wherein the polymer throughput is more than 25 grams per minute per orifice.

8. The hygiene article according to any one of claims 1 to 7, wherein the layer comprises at least 75% of nanofibers having a diameter of less than one micron.

9. The hygiene article according to any one of claims 1 to 8, wherein the polymer is a polyolefin and the polymer temperature during the melt fibrillation process is less than 220°C.

10. The hygiene article according to any one of claims 1 to 9, wherein the nanofibers are produced by a melt film fibrillation process.

11. The hygiene article according to any one of claims 1 to 10, wherein the hygiene article is selected from the group consisting of diapers, training pants, adult incontinence pads, catamenials products such as feminine care pads and pantiliners, tampons, personal cleansing articles, personal care articles, and hygiene wipes such as baby wipes, facial wipes, and feminine wipes, and combinations thereof.

## Patentansprüche

1. Hygieneartikel, eine Vliesbahn umfassend, die eine Schicht umfasst, die mindestens 35 % Nanofasern mit einem Durchmesser von weniger als einem Mikrometer aufweist,
wobei die Nanofasern aus einem oder mehreren thermoplastischen Polymeren erzeugt worden sind und aus einem einstufigen Schmelzfibrillierungsprozess mit einem Polymerdurchsatz von mehr als 10 Gramm pro Minute pro Düse hergestellt worden sind, und
wobei für den Schmelzfibrillierungsprozess ein faserbildendes Fluid genutzt wird, das bei einer Temperatur in der Nähe von, bei oder unter der Temperatur des geschmolzenen Polymers bzw. der geschmolzenen Polymere zugeführt wird.

2. Hygieneartikel nach Anspruch 1, wobei das Polymer eine Schmelzflussrate von weniger als 400 Dezigramm pro Minute aufweist.

3. Hygieneartikel nach Anspruch 2, wobei das Polymer eine Schmelzflussrate von weniger als 100 Dezigramm pro Minute aufweist.

4. Hygieneartikel nach einem der Ansprüche 1 bis 3, wobei für den Schmelzfibrillierungsprozess ein faserbildendes Fluid genutzt wird, das bei einer Temperatur zugeführt wird, die unter dem Schmelzpunkt des Polymers, vorzugsweise um mehr als 50 °C unter dem Schmelzpunkt des Polymers liegt.

5. Hygieneartikel nach einem der Ansprüche 1 bis 4, wobei für den Schmelzfibrillierungsprozess ein faserbildendes Fluid verwendet wird, das bei Umgebungstemperatur zugeführt wird.

6. Hygieneartikel nach einem der Ansprüche 1 bis 5, wobei für den Schmelzfibrillierungsprozess ein faserbildendes Fluid mit einer Geschwindigkeit von weniger als 100 Meter pro Sekunde verwendet wird.

7. Hygieneartikel nach einem der Ansprüche 1 bis 6, wobei der Polymerdurchsatz mehr als 25 Gramm pro Minute pro Düse beträgt.

8. Hygieneartikel nach einem der Ansprüche 1 bis 7, wobei die Schicht mindestens 75 % Nanofasern mit einem Durchmesser von weniger als einem Mikrometer umfasst.

9. Hygieneartikel nach einem der Ansprüche 1 bis 8, wobei das Polymer ein Polyolefin ist und die Polymertemperatur während des Schmelzfibrillierungsprozesses unter 220 °C liegt.

10. Hygieneartikel nach einem der Ansprüche 1 bis 9, wobei die Nanofasern anhand eines Schmelzfilmfibrillierungsprozesses erzeugt werden.

11. Hygieneartikel nach einem der Ansprüche 1 bis 10, wobei der Hygieneartikel ausgewählt ist aus der Gruppe bestehend aus Windeln, Übungshöschen, Erwachsenen-Inkontinenzeinlagen, Produkten für die Damenhygiene, wie Damenbinden und Slipeinlagen, Tampons, Körperreinigungsartikeln, Körperpflegeartikeln und Hygienetüchern, wie Babytüchern, Gesichtstüchern, Körperreinigungstüchern und Reinigungstüchern für die weibliche Intimhygiene und deren Kombinationen.

## Revendications

1. Article hygiénique comprenant une toile non tissée comprenant une couche contenant au moins 35% de nanofibres présentant un diamètre inférieur à un micron,
lesdites nanofibres étant produites à partir d'un ou de plusieurs polymères thermoplastiques et par un procédé de fibrillation en masse fondue à une étape présentant un débit en polymère supérieur à 10 grammes par minute et par orifice ; et
le procédé de fibrillation en masse fondue utilisant un fluide de défibrage alimenté à une température proche de, égale ou inférieure à la température du ou des polymère(s) fondu(s).

2. Article hygiénique selon la revendication 1, le polymère présentant un indice de fluidité inférieur à 400 décigrammes par minute.

3. Article hygiénique selon la revendication 2, le polymère présentant un indice de fluidité inférieur à 100 décigrammes par minute.

4. Article hygiénique selon l'une quelconque des revendications 1 à 3, le procédé de fibrillation en masse fondue utilisant un fluide de défibrage alimenté à une température inférieure au point de fusion du polymère, de préférence inférieure de plus de 50°C au point de fusion du polymère.

5. Article hygiénique selon l'une quelconque des revendications 1 à 4, le procédé de fibrillation en masse fondue utilisant un fluide de défibrage alimenté à température ambiante.

6. Article hygiénique selon l'une quelconque des revendications 1 à 5, le procédé de fibrillation en masse fondue utilisant un fluide de défibrage doté d'une vitesse inférieure à 100 mètres par seconde.

7. Article hygiénique selon l'une quelconque des revendications 1 à 6, le débit du polymère étant supérieur à 25 grammes par minute et par orifice.

8. Article hygiénique selon l'une quelconque des revendications 1 à 7, la couche comprenant au moins 75% de nanofibres présentant un diamètre inférieur à un micron.

9. Article hygiénique selon l'une quelconque des revendications 1 à 8, le polymère étant une polyoléfine et la température du polymère pendant le procédé de fibrillation en masse fondue étant inférieure à 220 °C.

10. Article hygiénique selon l'une quelconque des revendications 1 à 9, les nanofibres étant produites par un procédé de fibrillation de film en masse fondue.

11. Article hygiénique selon l'une quelconque des revendication 1 à 10, l'article hygiénique étant choisi dans le groupe constitué de couches, culottes d'apprentissage à la propreté, serviettes de protection contre les fuites urinaires pour adultes, produits cataméniaux tels que tampons de soin féminin et protège-slips, tampons, articles de nettoyage corporel, articles de soin personnel et lingettes hygiéniques telles que des lingettes pour bébés, des lingettes pour le visage et des lingettes féminines, et leurs combinaisons.
